# EUROPEAN PATENT SPECIFICATION

(11) **EP 3 628 207 B1**
(45) Date of publication and mention of the grant of the patent: **18.05.2022**
(21) Application number: 19199588.5
(22) Date of filing: 25.09.2019
(51) Int. Cl.: A61B 1/00

(54) **SURGICAL SYSTEM AND METHOD OF DISPLAYING INFORMATION IN THE SAME**
CHIRURGISCHES SYSTEM UND VERFAHREN ZUR ANZEIGE VON INFORMATIONEN DARIN
SYSTÈME CHIRURGICAL ET PROCÉDÉ D'AFFICHAGE D'INFORMATIONS DANS LE SYSTÈME

(30) Priority: 25.09.2018 JP 2018179277
(43) Date of publication of application: 01.04.2020
(73) Proprietor: Medicaroid Corporation, Kobe-shi, Hyogo 650-0047 (JP)
(72) Inventor: ISHIHARA, Kazuki, Hyogo, 650-0047 (JP); TSURUMOTO, Kenichiro, Kyoto, 603-8053 (JP); SUZUKI, Yuki, Kyoto, 603-8053 (JP); KUNIMOTO, Masaya, Kyoto, 603-8053 (JP); KIMPARA, Yuki, Kyoto, 603-8053 (JP); KAGEYAMA, Yuichi, Kyoto, 603-8053 (JP)
(74) Representative: Müller Hoffmann & Partner

(56) References cited:
- WO-A1-2011/125007
- WO-A1-2018/096987
- WO-A1-2018/170031
- US-A1- 2010 228 264
- US-A1- 2014 171 959

## Description

### BACKGROUND

The disclosure may relate to a surgical system, and may particularly relate to a surgical system including a remote control apparatus for operating surgical instruments.

In a related art, there has been known a surgical system including a remote control apparatus for operating surgical instruments (e.g., see Document 1).

Document 1 discloses a surgical system that includes: manipulators that support an endoscope for capturing images of the surgery site and surgical instruments; a display device that displays the images captured by the endoscope; and a remote control apparatus that includes operation handles to remotely operate the surgical instruments and foot pedals to execute functions of the surgical instruments. The surgical system is configured to display information on a surgical instrument operated by a right hand-operation handle in a right end region of the display device, to display information on a surgical instrument operated by a left hand-operation handle in a left end region of the display device, and to display information of positions of the foot pedals in a central region of the display device.

Document 1: US Patent No. 8418073. Document WO 2018/170031 discloses a surgical system, comprising: manipulators that respectively support an endoscope, a first surgical instrument, and a second surgical instrument; a remote control apparatus that includes a display device that displays an image captured by the endoscope.

### SUMMARY

The invention is disclosed in the appended set of claims. In the surgical system, although the operator can recognize arrangement of the foot pedals based on display of the foot pedal arrangement on the display device, there may be a problem that the operator cannot confirm that the foot is located on actually which foot pedal.

An object of one or more embodiments of the disclosure may be to provide a surgical system that is capable of confirming a foot pedal to be operated while keeping eyes on a display device.

An aspect of one or more embodiments may be a surgical system that includes: manipulators that respectively support an endoscope, a first surgical instrument, and a second surgical instrument; a remote control apparatus that includes a display device that displays an image captured by the endoscope, a first operation handle to operate the first surgical instrument, a second operation handle to operate the second surgical instrument, and an operation pedal section including foot pedals; and a control apparatus that controls at least the display device. The operation pedal section includes sensors that detect setting of a foot of an operator at an operation preparation position on the respective foot pedals. The foot pedals include a first foot pedal to execute a function of the first surgical instrument, a second foot pedal to execute a function of the second surgical instrument, and a third foot pedal to execute a function of the endoscope. The control apparatus displays a graphical user interface, overlapped with the image captured by the endoscope, on the display device, the graphical user interface including a first area that displays information on the first surgical instrument, a second area that displays information on the second surgical instrument, and a third area that displays information on the endoscope which are arranged therein. The control apparatus changes, when the foot of the operator is detected at the operation preparation position of at least one of the first to third foot pedals by at least one of the sensors, a display form of corresponding at least one of the first to third areas.

According to the aspect, the above-described configuration makes it possible to display the graphical user interface corresponding to the foot pedal with the display form of the graphical user interface changed, when the foot of the operator is set at the operation preparation position of the foot pedal.

According to the invention, it may be possible to provide a surgical system that is capable of confirming a foot pedal to be operated while keeping eyes on a display device.

### BRIEF DESCRIPTION OF DRAWINGS

FIG. 1 is a diagram illustrating an overview of a surgical system according to an embodiment;
FIG. 2 is a block diagram illustrating a view of a control-related configuration of the surgical system according to an embodiment;
FIG. 3 is a diagram illustrating a perspective view of an operation pedal section of a remote control apparatus according to an embodiment;
FIG. 4 is a diagram illustrating a plan view of the operation pedal section of the remote control apparatus according to an embodiment;
FIG. 5 is a diagram illustrating a front view of the operation pedal section of the remote control apparatus according to an embodiment;
FIG. 6 is a diagram illustrating views for explaining assignment examples of the operation pedal section of the remote control apparatus according to an embodiment;
FIG. 7 is a diagram illustrating a view of an example of a normal screen displayed on a display device according to an embodiment;
FIG. 8 is a diagram illustrating a view of an example of a screen of the display device when a switch pedal is operated according to an embodiment;
FIG. 9 is a diagram illustrating a view of an example of a screen of the display device when a foot is at an operation preparation position for a clutch pedal according to an embodiment;
FIG. 10 is a diagram illustrating a view of an example of a screen of the display device when the clutch pedal is operated according to an embodiment;
FIG. 11 is a diagram illustrating a view of an example of a screen of the display device when a clutch switch (right) is operated according to an embodiment;
FIG. 12 is a diagram illustrating a view of an example of a screen of the display device when a clutch switch (left) is operated according to an embodiment;
FIG. 13 is a diagram illustrating a view of an example of a screen of the display device when a foot is at an operation preparation position for a camera pedal according to an embodiment;
FIG. 14 is a diagram illustrating a view of an example of a screen of the display device when the camera pedal is operated according to an embodiment;
FIG. 15 is a diagram illustrating a view of an example of a screen of the display device when a foot is at an operation preparation position for a cutting pedal (right) according to an embodiment;
FIG. 16 is a diagram illustrating a view of an example of a screen of the display device when the cutting pedal (right) is operated according to an embodiment;
FIG. 17 is a diagram illustrating a view of an example of a screen of the display device when a foot is at an operation preparation position for a coagulation pedal (right) according to an embodiment;
FIG. 18 is a diagram illustrating a view of an example of a screen of the display device when the coagulation pedal (right) is operated according to an embodiment;
FIG. 19 is a diagram illustrating a view of an example of a screen of the display device when feet are at operation preparation positions for a cutting pedal (left) and the clutch pedal according to an embodiment;
FIG. 20 is a diagram illustrating a view of an example of a screen of the display device on which a function limit notification is displayed according to an embodiment; and
FIG. 21 is a diagram illustrating a view of an example of a screen of the display device on which an error notification is displayed according to an embodiment.

### DETAILED DESCRIPTION

Descriptions are provided hereinbelow for one or more embodiments based on the drawings. In the respective drawings referenced herein, the same constituents are designated by the same reference numerals and duplicate explanation concerning the same constituents is omitted. All of the drawings are provided to illustrate the respective examples only.

### (Configuration of Surgical System)

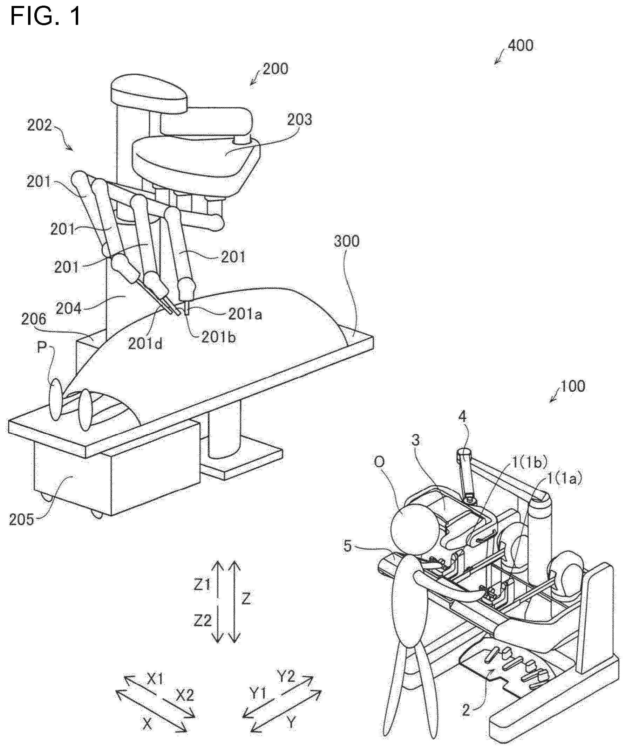

A configuration of a surgical system 400 according to one or more embodiments is described with reference to FIGS. 1 to 21.

As illustrated in FIG. 1, the surgical system 400 includes a remote control apparatus 100 and a patient-side apparatus 200. The remote control apparatus 100 is provided for teleoperation of medical equipment included in the patient-side apparatus 200. When an operator O, as a surgeon, inputs an action mode instruction to be executed by the patient-side apparatus 200 to the remote control apparatus 100, the remote control apparatus 100 transmits the action mode instruction to the patient-side apparatus 200 through a controller 206. In response to the action mode instruction transmitted from the remote control apparatus 100, the patient-side apparatus 200 operates medical equipment, such as surgical instruments 201a to 201c and an endoscope 201d, attached to manipulators 201. This allows for minimally invasive surgery.

The patient-side apparatus 200 constitutes an interface to perform a surgery for a patient P. The patient-side apparatus 200 is placed beside an operation table 300 on which the patient P lies. The patient-side apparatus 200 includes the manipulators 201. One of the manipulators 201 supports the endoscope 201d while the others hold the surgical instruments 201a to 201c. The manipulators 201 are commonly supported by a platform 203. Each of the manipulators 201 includes joints. Each joint includes a driver including a servo-motor and a position detector such as an encoder. The manipulators 201 are configured so that the medical equipment attached to each manipulator 201 is controlled by a driving signal given through the controller 206, to perform a desired movement.

The platform 203 is supported by a positioner 202 placed on the floor of an operation room. The positioner 202 includes a column 204 and a base 205. The column 204 includes an elevating shaft adjustable in the vertical direction. The base 205 includes wheels and is movable on the floor surface.

The surgical instruments 201a to 201c as the medical equipment are detachably attached to the tip of three of the four manipulators 201, respectively. The surgical instruments 201a to 201c each include a housing, a shaft, and an end effector. The housing is attached to the corresponding one of the manipulators 201. The shaft is in elongated shape. The end effector is provided at the tip of the shaft. The end effector is grasping forceps, scissors, a hook, a high-frequency knife, a snare wire, a clamp, or a stapler, for example. The end effector is not limited to those and can be various types of treatment tools. In surgeries using the patient-side apparatus 200, the three manipulators 201 introduce the surgical instruments 201a to 201c into the body of the patient P through a cannula (trocar) placed on the body surface of the patient P. The end effector of each of the surgical instruments 201a to 201c is then located near the surgery site. The surgical instrument 201a, the surgical instrument 201b, and the surgical instrument 201c are examples of a first surgical instrument, a second surgical instrument, and a replacement surgical instrument of Claims, respectively.

To the tip of one of the four manipulators 201, the endoscope 201d as the medical equipment is detachably attached. The endoscope 201d captures an image within the body cavity of the patient P. The captured image is outputted to the remote control apparatus 100. The endoscope 201d is a 3D endoscope capable of capturing a three-dimensional image or a 2D endoscope. In surgeries using the patient-side apparatus 200, the one of the manipulators 201 introduces the endoscope 201d into the body of the patient P through a trocar placed on the body surface of the patient P. The endoscope 201d is then located near the surgery site.

The remote control apparatus 100 constitutes the interface with the operator O. The remote control apparatus 100 is an apparatus that allows the operator O to operate the medical equipment held by the manipulators 201. Specifically, the remote control apparatus 100 is configured to transmit action mode instructions which are inputted by the operator O and are to be executed by the surgical instruments 201a to 201c and endoscope 201d, to the patient-side apparatus 200 through the controller 206. The remote control apparatus 100 is installed beside the operation table 300 so that the operator O can see the state of the patient P very well while operating operation handles 1, for example. The remote control apparatus 100 may be configured to transmit the action mode instructions wirelessly and installed in a room different from the operation room where the operation table 300 is installed.

The action modes to be executed by the surgical instruments 201a to 201c include a mode of actions to be taken by each of the surgical instruments 201a to 201c (a series of positions and postures) and actions to be executed by the function of each of the surgical instruments 201a to 201c. For one of the surgical instruments 201a to 201c which is a pair of grasping forceps, for example, the action mode to be executed by the surgical instruments 201a to 201c includes roll and pitch positions of the wrist of the end effector and the action to open or close the jaws. For one of the surgical instruments 201a to 201c which is a high-frequency knife, the action mode to be executed by the surgical instruments 201a to 201c includes vibration of the high-frequency knife, specifically, supply of current to the high-frequency knife. For one of the surgical instruments 201a to 201c which is a snare wire, the action mode to be executed by the surgical instruments 201a to 201c includes a capturing action and an action to release the captured object and moreover includes an action to supply current to a bipolar or monopolar instrument to burn off the surgery site.

The action mode to be executed by the endoscope 201d includes setting of the position and posture of the tip of the endoscope 201d or setting of the zoom magnification, for example.

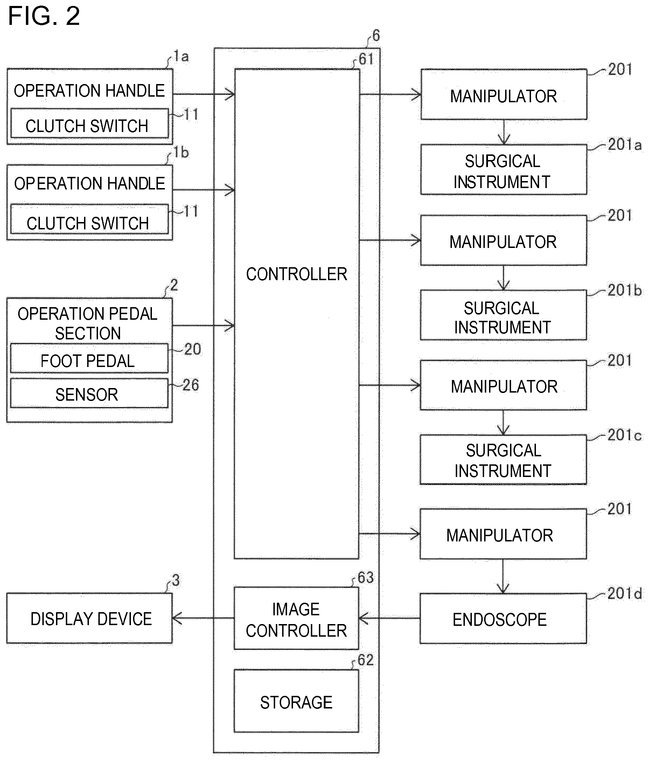

As illustrated in FIGS. 1 and 2, the remote control apparatus 100 includes operation handles 1, an operation pedal section 2, a display device 3, a display device supporting arm 4 supporting the display device 3, an armrest 5 supporting the arms of the operator O, and a control apparatus 6.

The operation handles 1 are provided in order to remotely operate the medical equipment held by the manipulators 201. Specifically, the operation handles 1 accept operations by the operator O for operating the medical equipment (the surgical instruments 201a to 201c and the endoscope 201d). The operation handles 1 include a pair of operation handles 1 arranged side by side in the X direction. The operation handles 1 includes an operation handle 1a, which is arranged on the right side (the X2 side) to be operated by the right hand of the operator O, and an operation handle 1b, which is arranged on the left side (the X1 side) to be operated by the left hand of the operator O. The operation handle 1a and the operation handle 1b are examples of a first operation handle and a second operation handle of Claims, respectively.

The operation handles 1 are arranged to extend from the rear side (the Y2 side) of the remote control apparatus 100 to the front side (the Y1 side) thereof. The operation handles 1 are configured to be movable within a predetermined three-dimensional operation region. Specifically, the operation handles 1 are configured to be movable in the up-down direction (the Z direction), the right-left direction (the X direction), and the front-rear direction (the Y direction).

The right hand-operation handle 1a is provided to remotely operate the corresponding surgical instrument 201a. The left hand-operation handle 1b is provided to remotely operate the corresponding surgical instrument 201b. As illustrated in FIG. 2, a clutch switch 11 is provided in each of the operation handles 1a and 1b. The clutch switch 11 is operated when temporarily disconnecting the control-related connections between the manipulators 201 and the operation handles 1a and 1b. When the clutch switch 11 of the operation handle 1a is operated, the control-related connection between the operation handle 1a and one of the manipulators 201 to which the surgical instrument 201a is provided is temporarily disconnected. When the clutch switch 11 of the operation handle 1b is operated, the control-related connection between the operation handle 1b and another one of the manipulators 201 to which the surgical instrument 201b is provided is temporarily disconnected.

The remote control apparatus 100 and patient-side apparatus 200 constitute a master-slave system in terms of controlling motion of the manipulators 201. Specifically, the operation handles 1 constitute an operating section on the master side in the master-slave system, and the manipulators 201 supporting the medical equipment constitute an operating section on the slave side. When the operator O operates the operation handles 1, the motion of the manipulators 201 is controlled so that the tips (the end effectors of the surgical instruments 201a to 201c or the endoscope 201d) of the manipulators 201 move following the movement of the operation handles 1.

The patient-side apparatus 200 is configured to control the motion of the manipulators 201 in accordance with the set motion scaling ratio. When the motion scaling ratio is set to 1/2, for example, the end effectors of the surgical instruments 201a to 201c move 1/2 of the movement distance of the operation handles 1. This allows for precise fine surgery.

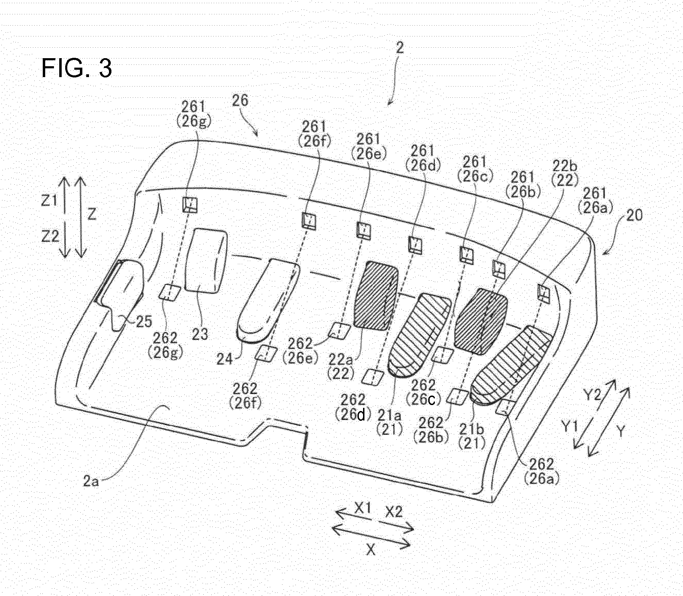

As illustrated in FIGS. 3 to 5, the operation pedal section 2 includes foot pedals 20 that execute functions of the medical equipment. The foot pedals 20 are arranged on a base 2a. The foot pedals 20 include coagulation pedals 21, cutting pedals 22, a clutch pedal 23, and a camera pedal 24. Additionally, the operation pedal section 2 is provided with a switch pedal 25. The coagulation pedals 21, cutting pedals 22, clutch pedal 23, and camera pedal 24 are operated by being pressed downward. The switch pedal 25 is operated by being pressed in the horizontal direction. The foot pedals 20 are operated by the foot of the operator O.

The operation pedal section 2 includes sensors 26 that detect the presence of the foot operating the foot pedals 20. The sensors 26 include sensors 26a, 26b, 26c, 26d, 26e, 26f, and 26g. The sensors 26 each include an emitter 261 and a receiver 262. The sensors 26 detect the presence of the foot when the light from the emitter 261 is blocked by the foot and the light reception by the receiver 262 is interrupted. Specifically, the sensors 26 are blockage type sensors. The sensors 26 are provided to detect that the foot of the operator O is put on any one of the foot pedals 20. In other words, the sensors 26 detect setting of the foot of the operator O at an operation preparation position of the foot pedal 20.

The detection information detected by the sensors 26 is transmitted to a controller 61, and the controller 61 that receives the detection information determines whether there is the foot operating the corresponding foot pedals 20.

The coagulation pedals 21 enable surgical instruments to coagulate surgery sites. Specifically, when the coagulation pedals 21 are operated, voltage for coagulation is applied to corresponding one of the surgical instruments 201a, 201b and 201c to coagulate surgery sites. The coagulation pedals 21 include a coagulation pedal 21a and a coagulation pedal 21b. The coagulation pedal 21a is located to the left (on the X1 side) of the coagulation pedal 21b. The coagulation pedal 21a is used in relation to the surgical instrument 201b controlled by the left hand-operation handle 1b. The coagulation pedal 21b is used in relation to the surgical instrument 201a controlled by the right hand-operation handle 1a. The coagulation pedals 21a and 21b are in a first color. For example, the coagulation pedals 21a and 21b are in blue as the first color.

The cutting pedals 22 enable the surgical instruments to cut surgery sites. Specifically, when the cutting pedals 22 are operated, voltage for cutting is applied to corresponding one of the surgical instruments 201a, 201b, and 201c to cut surgery sites. The cutting pedals 22 include a cutting pedal 22a and a cutting pedal 22b. The cutting pedal 22a is located to the left (on the X1 side) of the cutting pedal 22b. The cutting pedal 22a is used in relation to the surgical instrument 201b controlled by the left hand-operation handle 1b. The cutting pedal 22b is used in relation to the surgical instrument 201a controlled by the right hand-operation handle 1a. The cutting pedals 22a and 22b are in a second color different from the first color. For example, the cutting pedals 22a and 22b are in yellow as the second color.

Specifically, the right-side foot pedals (the coagulation pedal 21b and the cutting pedal 22b) execute the function of the surgical instrument 201a operated by the right hand-operation handle 1a. The left-side foot pedals (the coagulation pedal 21a and the cutting pedal 22a) execute the function of the surgical instrument 201b operated by the left hand-operation handle 1b. The coagulation pedal 21b is an example of a first foot pedal and a first coagulation pedal of Claims, and the cutting pedal 22b is an example of the first foot pedal and a first cutting pedal of Claims. The coagulation pedal 21a is an example of a second foot pedal and a second coagulation pedal of Claims, and the cutting pedal 22a is an example of the second foot pedal and a second cutting pedal of Claims.

The clutch pedal 23 is operated to execute a clutch function. Specifically, the clutch pedal 23 is used when temporarily disconnecting the control-related connections between the manipulators 201 and the operation handles 1a and 1b to stop the operation of the surgical instruments. During the operation of the clutch pedal 23, the manipulators 201 of the patient-side apparatus 200 does not move even when the operation handles 1a and 1b are operated. For example, when the operation handles 1 come close to the end of the movable region during the operation, it is possible to move the operation handles 1 back to near the central position by operating the clutch pedal 23 and temporarily disconnecting the control-related connections. Then, once the operation of the clutch pedal 23 is quitted, the manipulators 201 and the operation handles 1 are connected again, and operation of the operation handles 1 can be restarted from near the central position. In this case, the clutch pedal 23 is operated to temporarily disconnect the whole control-related connections between both the operation handles 1a and 1b and the manipulators 201. On the other hand, the clutch switch 11 provided in each of the operation handles 1a and 1b is operated to temporarily disconnect the control-related connections between either of the operation handles 1a and 1b and corresponding one of the manipulators 201, individually. The clutch pedal 23 is an example of a fifth foot pedal of Claims.

The camera pedal 24 is used to control the position and orientation of the endoscope 201d that captures images within the body cavity. That is, the camera pedal 24 executes a function of the endoscope 201d. Specifically, the camera pedal 24 enables control of the endoscope 201d by the operation handles 1. That is, the position and orientation of the endoscope 201d are controllable by the operation handles 1 while the camera pedal 24 is being pressed. The endoscope 201d is controlled by using both of the right and left operation handles 1, for example. Specifically, when the operator O rotates the right and left operation handles 1 about the middle point between the right and left operation handles 1, the endoscope 201d is rotated. When the operator O presses the right and left operation handles 1 together, the endoscope 201d goes further into the body cavity. When the operator O pulls the right and left operation handles 1 together, the endoscope 201d retracts. When the operator O moves the right and left operation handles 1 together up, down, right, and left, the endoscope 201d moves up, down, right, and left, respectively. The camera pedal 24 is an example of a third foot pedal of Claims.

The switch pedal 25 is used to switch the manipulators 201 to be operated by the operation handles 1. For example, four manipulators 201 are provided, and within three of the manipulators 201 respectively holding the surgical instruments 201a to 201c, the manipulators 201 operated by the right and left operation handles 1 are switched by operating the switch pedal 25. Specifically, the switch pedal 25 is operated to replace the surgical instrument 201a operated by the right hand-operation handle 1a or the surgical instrument 201b operated by the left hand-operation handle 1b with the surgical instrument 201c, which is provided for replacement. The switch pedal 25 is operated by being pressed leftward (in the X1 direction). For example, the manipulator 201 operated by the right hand-operation handle 1a is switched by operating the switch pedal 25. In this case, the manipulator 201 operated by the right hand-operation handle 1a is switched without changing the manipulator 201 operated by the left hand-operation handle 1b. For example, the manipulator 201 operated by the left hand-operation handle 1b is switched by operating the switch pedal 25. In this case, the manipulator 201 operated by the left hand-operation handle 1b is switched without changing the manipulator 201 operated by the right hand-operation handle 1a. The switch pedal 25 is an example of a fourth foot pedal of Claims.

As illustrated in FIGS. 3 to 5, the foot pedals 20 are arranged from the left side (the X1 side) to the right side (the X2 side) in order of the switch pedal 25, the clutch pedal 23, the camera pedal 24, the cutting pedal 22a, the coagulation pedal 21a, the cutting pedal 22b, and the coagulation pedal 21a.

The arrangement of the foot pedals 20 illustrated in FIGS. 3 to 5 is suitable for operating the pairs of coagulation pedals 21 and cutting pedals 22 assigned to the respective right and left operation handles 1 with only the right foot. The pedals arrangement may be as follows: the pair of coagulation pedal 21a and cutting pedal 22a is located to the left (the X1 side) of the clutch pedal 23 and camera pedal 24, and the clutch pedal 23 and camera pedal 24 are located at the center (between the pair of coagulation pedal 21a and cutting pedal 22a and the pair of coagulation pedal 21b and cutting pedal 22b). This arrangement is suitable for operating the coagulation pedal 21a and cutting pedal 22a assigned to the left hand-operation handle 1b with the left foot while operating the coagulation pedal 21b and cutting pedal 22b assigned to the right hand-operation handle 1a with the right foot.

The base 2a on which the foot pedals 20 are arranged is movable in the horizontal direction. Specifically, two sides in the axial direction (the X direction) of the base 2a are connected with a base of the remote control apparatus 100 with sliding bearings, and the operation pedal section 2 is slidable and movable in the depth direction (the front-rear direction, the Y direction). The operation pedal section 2 can be moved in the depth direction electrically by a driver device such as a motor provided in the base of the remote control apparatus 100. This makes it possible to adjust the positions of the foot pedals 20 depending on the operation posture, physique, or preference of the operator O.

The number of the sensors 26 that detect the presence of the foot is greater than the number of the foot pedals 20 that are operated by being pressed downward. Specifically, the number of the provided sensors 26 is seven. On the other hand, the number of the provided foot pedals 20 operated by being pressed downward is six (the coagulation pedals 21a and 21b, cutting pedals 22a and 22b, clutch pedal 23, and camera pedal 24).

The sensors 26 used to detect the foot moved for the operation are provided near one of the foot pedals 20, and the sensors 26 are used to determine whether there is the foot that is operating or that is trying to operate the one of the foot pedals 20. Specifically, when the controller 61 receives the detection information from the sensors 26 located near the one of the foot pedals 20, the controller 61 determines that the foot of the operator O is set at the operation preparation position of the one of the foot pedals 20.

In order to simplify the descriptions, in this specification, a configuration in which one of the sensors 26 (for emitter-receiver type sensors, that is straight light emitted from an emitter) is positioned between a central section of a tip side (the Y1 side) of one foot pedal 20 and a central section of a tip side (the Y1 side) of another foot pedal 20 next to the one foot pedal 20 is expressed simply as "a sensor is positioned between one pedal and another pedal next to the one pedal." In addition, in this specification, the sensor 26 positioned in a central section of a tip side (the Y1 side) of one foot pedal 20 (for the emitter-receiver type sensors, that is a sensor including an emitter that emits straight light passing through the central section of the tip side of the one foot pedal 20) and a sensor between the one pedal and another pedal next to the one pedal are each defined as "a sensor arranged near one pedal."

The sensors 26a and 26b detect the foot coming closer to the coagulation pedal 21b. Specifically, when both the sensors 26a and 26b detect the foot, the controller 61 determines that the foot pedal 20 to be operated is the coagulation pedal 21b. The sensors 26b and 26c detect the foot coming closer to the cutting pedal 22b. Specifically, when both the sensors 26b and 26c detect the foot, the controller 61 determines that the foot pedal 20 to be operated is the cutting pedal 22b.

The sensors 26c and 26d detect the foot coming closer to the coagulation pedal 21a. Specifically, when both the sensors 26c and 26d detect the foot, the controller 61 determines that the foot pedal 20 to be operated is the coagulation pedal 21a. The sensors 26d and 26e detect the foot coming closer to the cutting pedal 22a. Specifically, when both the sensors 26d and 26e detect the foot, the controller 61 determines that the foot pedal 20 to be operated is the cutting pedal 22a.

The sensor 26f detects the foot coming closer to the camera pedal 24. Specifically, when the sensor 26f detects the foot, the controller 61 determines that the foot pedal 20 to be operated is the camera pedal 24. The sensor 26g detects the foot coming closer to the clutch pedal 23. Specifically, when the sensor 26g detects the foot, the controller 61 determines that the foot pedal 20 to be operated is the clutch pedal 23.

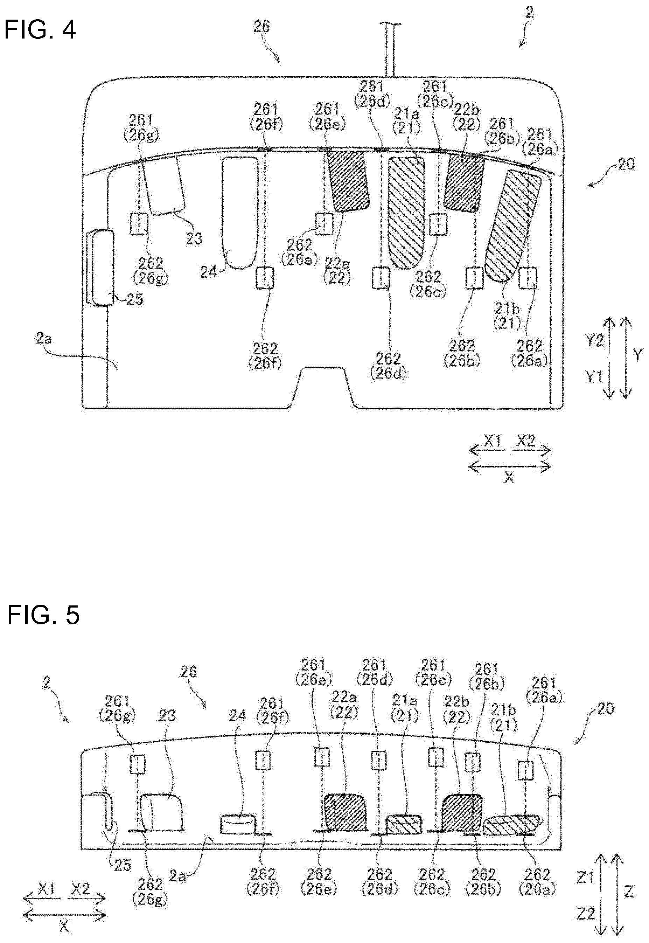

The emitter 261 includes a light-emitting element such as an LED. The emitter 261 is configured to emit visible light or invisible light such as infrared light. The receiver 262 includes a light-receiving element. The corresponding emitter 261 and receiver 262 are arranged along the Y direction in the plan view. Specifically, as illustrated as a broken line in FIGS. 3 and 5, the light from the emitter 261 is emitted substantially along the Y direction in the plan view. The corresponding emitter 261 and receiver 262 are arranged so that their arrangement orientation is parallel to each other in the plan view. The emitter 261 is configured to emit the light obliquely downward.

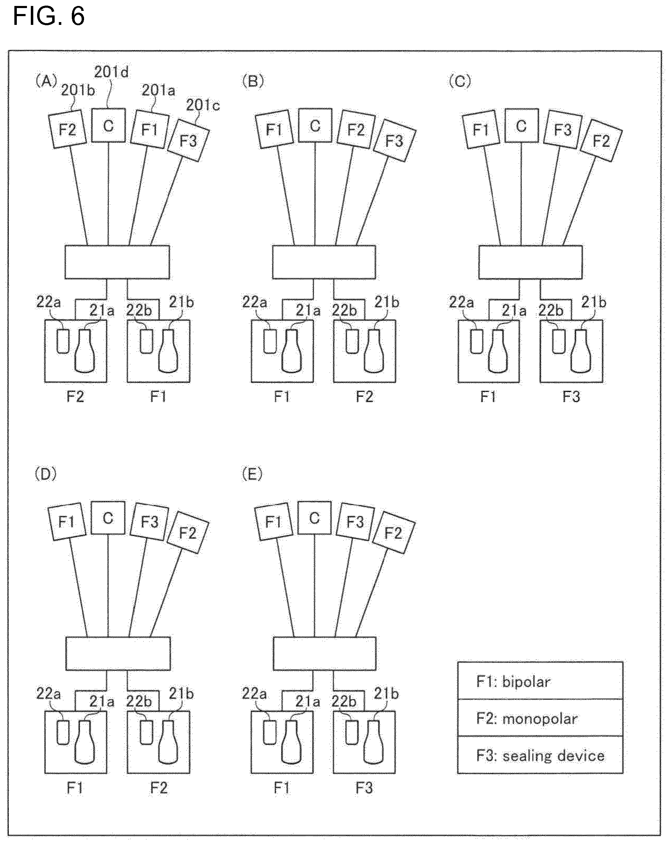

An assignment example of the coagulation pedals 21 (21a and 21b) and the cutting pedals 22 (22a and 22b) on the operation pedal section 2 is described with reference to FIG. 6. The coagulation pedal 21a and the cutting pedal 22a are used as a pair, while the coagulation pedal 21b and the cutting pedal 22b are used as a pair. In this case, it is possible to use a pair of forceps (e.g., a grasper) to cut and coagulate the surgery site. When a pair of forceps is used for cutting and coagulating, high voltage is applied for cutting, and voltage lower than the cutting case is applied for coagulating. Specifically, it is possible to coagulate and cut the surgery site by selecting and using the coagulation pedal 21a (21b) and the cutting pedal 22a (22b). Although a grasper and the like can be used for cutting and coagulating, a sealing device for coagulating may also be used as a dedicated tool or used concurrently with another tool. This is because the sealing device often has an additional function such as automatic termination of power supply that is executed when coagulating is completed.

In the example illustrated in FIG. 6, a pair of bipolar forceps F1, a pair of monopolar forceps F2, and a sealing device F3 as the medical equipment and the endoscope 201d are attached to the four manipulators 201. The positional relationship between the four manipulators 201 is recognized by the position detector provided for each manipulator. The positional relationship between the manipulators in the right-left direction is determined based on the positions thereof seen from the platform 203. In FIG. 6A, the pair of monopolar forceps F2 is located to the left of one of the manipulators 201 to which the endoscope 201d is attached, and the pair of bipolar forceps F1 and the sealing device F3 are located to the right of the manipulator 201 to which the endoscope 201d is attached in this order from left. In the assignment of the coagulation pedals 21 (21a and 21b) and cutting pedals 22 (22a and 22b), the manipulator 201 to which the surgical instrument 201b is attached is assigned to the left-side foot pedals (the coagulation pedal 21a and the cutting pedal 22a), and the manipulator 201 to which the surgical instrument 201a, which is a surgical instrument to the right of the manipulator 201 to which the surgical instrument 201b is attached, is assigned to right-side foot pedals (the coagulation pedal 21b and cutting pedal 22b). Specifically, in FIG. 6A, the pair of monopolar forceps F2 is assigned to the left-side foot pedals (the coagulation pedal 21a and the cutting pedal 22a), while the pair of bipolar forceps F1 is assigned to the right-side foot pedals (the coagulation pedal 21b and the cutting pedal 22b). When only two surgical instruments are attached, the manipulators 201 are assigned to the left-side foot pedals and the right-side foot pedals in order from the one on the leftmost side of the manipulator 201 to which the endoscope 201d is attached. When only one surgical instrument is attached, the manipulator 201 is assigned to the left-side foot pedals.

In FIG. 6B, the pair of bipolar forceps F1 and the pair of monopolar forceps F2 are replaced with each other by an assistant (a nurse, for example). In this case, the types of the surgical instruments 201a and 201b are specified when the surgical instruments 201a and 201b are attached to the manipulators 201. For example, the IC of the interface may store information including model numbers of the instruments. The pair of bipolar forceps F1 is assigned to the left-side foot pedals (the coagulation pedal 21a and the cutting pedal 22a). The pair of monopolar forceps F2 is assigned to the right-side foot pedals (the coagulation pedal 21b and the cutting pedal 22b).

In FIG. 6C, the pair of monopolar forceps F2 and the sealing device F3 are replaced with each other by an assistant (a nurse, for example). In this case, the types of the surgical instruments 201a and 201b are specified when the surgical instruments 201a and 201b are attached to the manipulators 201. The pair of bipolar forceps F1 continues to be assigned to the left-side foot pedals (the coagulation pedal 21a and the cutting pedal 22a). The sealing device F3 is assigned to the right-side foot pedals (the coagulation pedal 21b and the cutting pedal 22b).

In FIG. 6D, the switch pedal 25 is operated to change which of the two manipulators 201 located on the right side is being activated. Specifically, the manipulators 201 controlled by the operation handles 1 are switched. The manipulator 201 to which the pair of bipolar forceps F1 is attached is controlled with the left hand-operation handle 1b while the manipulator 201 to which the pair of monopolar forceps F2 is attached is controlled with the right hand-operation handle 1a. The pair of bipolar forceps F1 continues to be assigned to the left-side foot pedals (the coagulation pedal 21a and the cutting pedal 22a). The pair of monopolar forceps F2 is assigned to the right-side foot pedals (the coagulation pedal 21b and the cutting pedal 22b).

In FIG. 6E, the switch pedal 25 is operated to change which of the two manipulators 201 on the right side is being activated. Specifically, the manipulators 201 to be controlled by the operation handles 1 are switched. The manipulator 201 to which the pair of bipolar forceps F1 is attached is controlled with the left hand-operation handle 1b while the manipulator 201 to which the sealing device F3 is attached is controlled with the right hand-operation handle 1a. The pair of bipolar forceps F1 continues to be assigned to the left-side foot pedals (the coagulation pedal 21a and the cutting pedal 22a). The sealing device F3 is assigned to the right-side foot pedals (the coagulation pedal 21b and the cutting pedal 22b).

In some cases, a simple grasper which works without electricity is used when strong grip is mainly necessary. Such surgical instruments that cannot be supplied with current are not controlled with the coagulation pedals 21 (21a and 21b) and the cutting pedals 22 (22a and 22b) and are therefore not assigned to the coagulation pedals 21 and cutting pedals 22. The assignment of the coagulation pedals 21 (21a and 21b) and cutting pedals 22 (22a and 22b) is configured so that the manipulator 201 holding a surgical instrument that cannot be supplied with current is ignored.

The left-side foot pedals (the coagulation pedal 21a and the cutting pedal 22a) and right-side foot pedals (the coagulation pedal 21b and the cutting pedal 22b) may be assigned by another rule. For example, the manipulators 201 to the right and left of the manipulator 201 to which the endoscope 201d is attached may be always assigned to the foot pedals. For example, the one manipulator 201 to the left of the manipulator 201 to which the endoscope 201d is attached may be assigned to the left-side foot pedals (the coagulation pedal 21a and the cutting pedal 22a), while the left manipulator 201 among the two manipulators 201 located to the right of the manipulator 201 to which the endoscope 201d is attached is assigned to the right-side foot pedals (the coagulation pedal 21b and the cutting pedal 22b).

As illustrated in FIG. 1, the display device 3 displays an image captured by the endoscope 201d. The display device 3 includes a scope type display device or a non-scope type display device. In the example illustrated in FIG. 1, the display device 3 is the scope type display device. The scope type display device is a display unit that the operator O looks into, for example. The non-scope type display device is a concept including an open-type display unit that the operator O looks at without looking into and that has a flat screen, such as a normal personal computer display.

As illustrated in FIG. 2, the control apparatus 6 such as a computer includes a controller 61, a storage 62, and an image controller 63, for example. The controller 61 includes a computing element such as a CPU. The storage 62 includes one or more memories, such as a ROM and a RAM. The control apparatus 6 is configured to control at least the endoscope 201d and the display device 3. The control apparatus 6 may be composed of a single controller performing centralized control or may be composed of plural controllers that perform decentralized control in cooperation with each other. The controller 61 determines whether the action mode instruction inputted by the operation handles 1 is to be executed by the surgical instruments 201a to 201c or to be executed by the endoscope 201d, depending on the state of the operation pedal section 2. The controller 61 transmits a signal to operate a corresponding one of the manipulators 201 based on the determination result and operations through the operation handles 1.

The controller 61 receives the detection information on the presence of the foot of the operator O from the sensors 26 and determines whether there is the foot of the operator O. When it is determined that there is the foot of the operator O, the controller 61 controls the display device 3 to display the corresponding screen.

The storage 62 stores control programs corresponding to the types of the surgical instruments 201a to 201c, for example. The controller 61 reads the stored control programs according to the types of the attached surgical instruments 201a to 201c. The action mode instructions from the operation handles 1 and/or the operation pedal section 2 of the remote control apparatus 100 thereby causes the respective surgical instruments 201a to 201c to perform proper motions.

The image controller 63 transmits an image acquired by the endoscope 201d to the display device 3. The image controller 63 modifies the image if necessary.

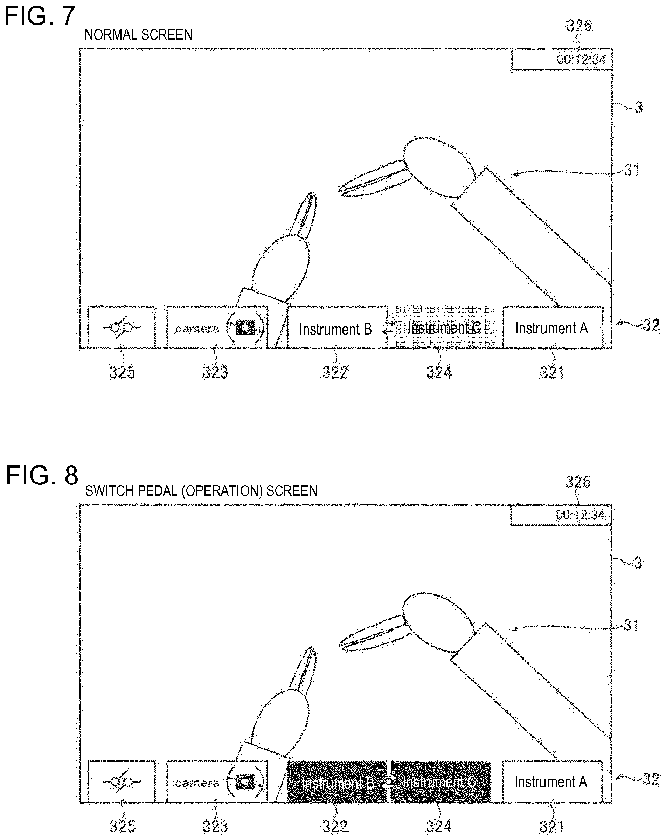

As illustrated in FIG. 7, the control apparatus 6 is configured to display a graphical user interface 32 on the display device 3 while overlapping the graphical user interface 32 on an image 31 captured by the endoscope 201d. The graphical user interface 32 includes a first area 321 showing information on the surgical instrument 201a operated by the right hand-operation handle 1a, a second area 322 showing information on the surgical instrument 201b operated by the left hand-operation handle 1b, and a third area 323 showing information on the endoscope 201d, which are arranged side by side in this order from right to left.

The control apparatus 6 is configured to display the graphical user interface 32 on the display device 3 while overlapping the graphical user interface 32 on the image 31 captured by the endoscope 201d. The graphical user interface 32 includes the first area 321 showing the information on the surgical instrument 201a operated by the right hand-operation handle 1a, the second area 322 showing the information on the surgical instrument 201b operated by the left hand-operation handle 1b, and the third area 323 showing the information on the endoscope 201d. The first area 321, second area 322, and third area 323 are arranged in the same order as the arrangement order of the right-side foot pedals (the coagulation pedal 21b and the cutting pedal 22b), left-side foot pedals (the coagulation pedal 21a and the cutting pedal 22a), and camera pedal 24 on the operation pedal section 2.

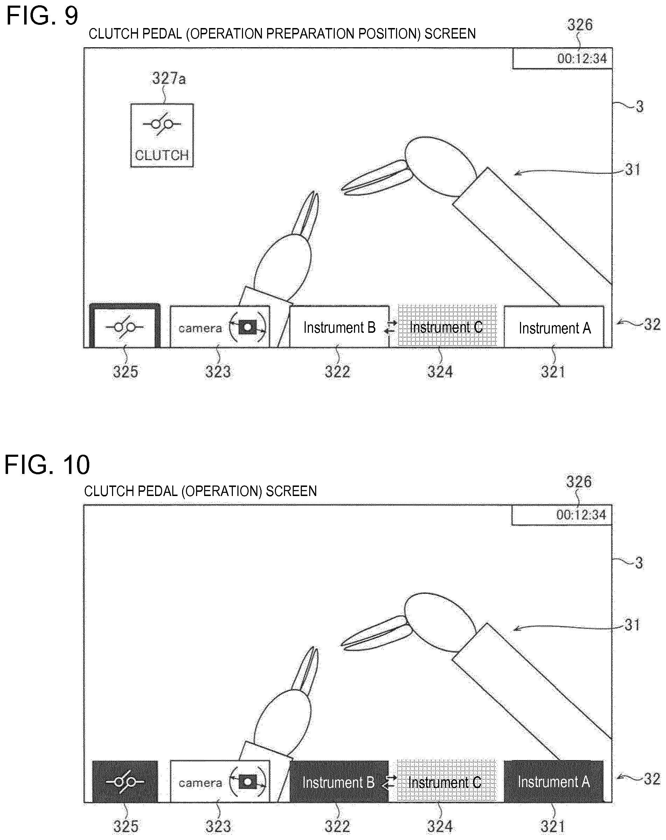

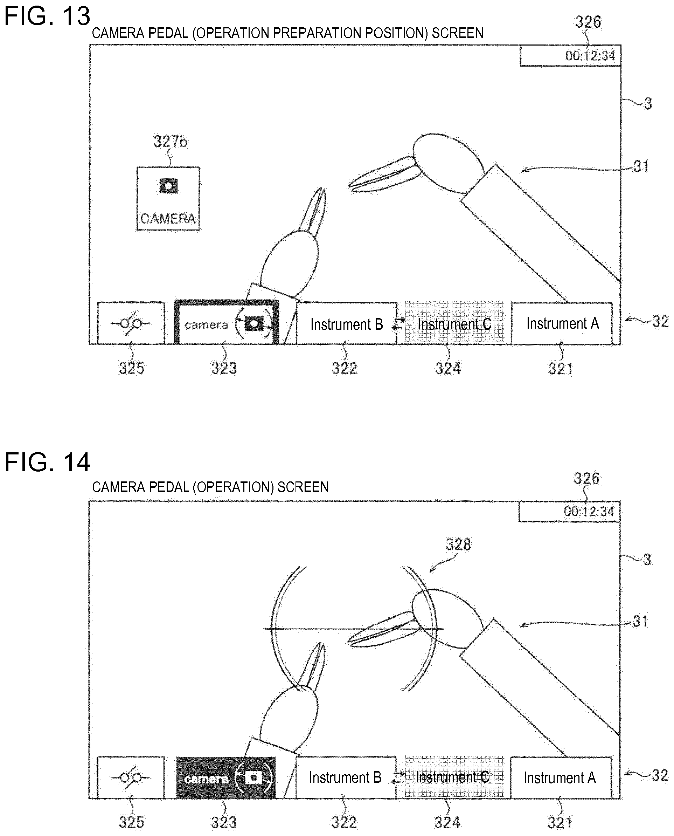

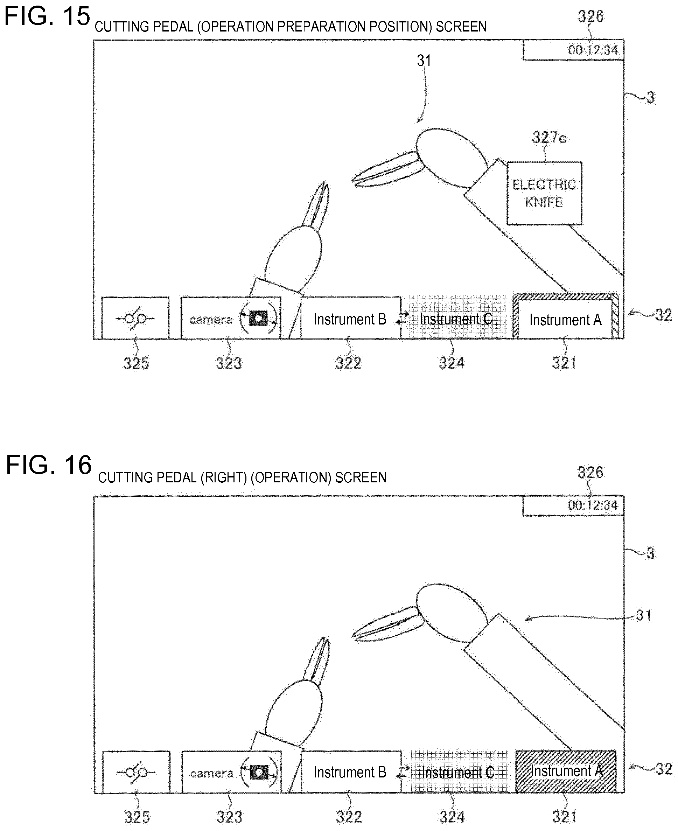

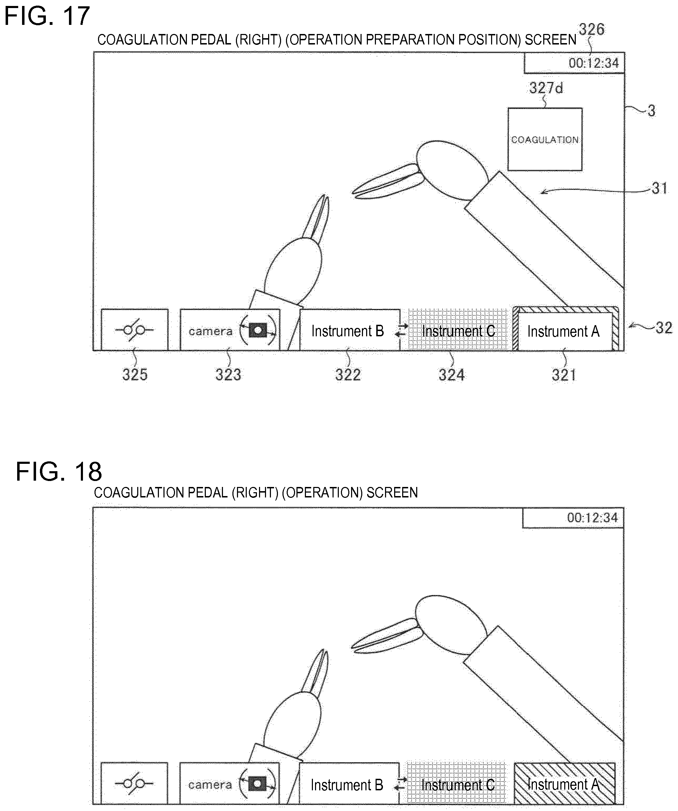

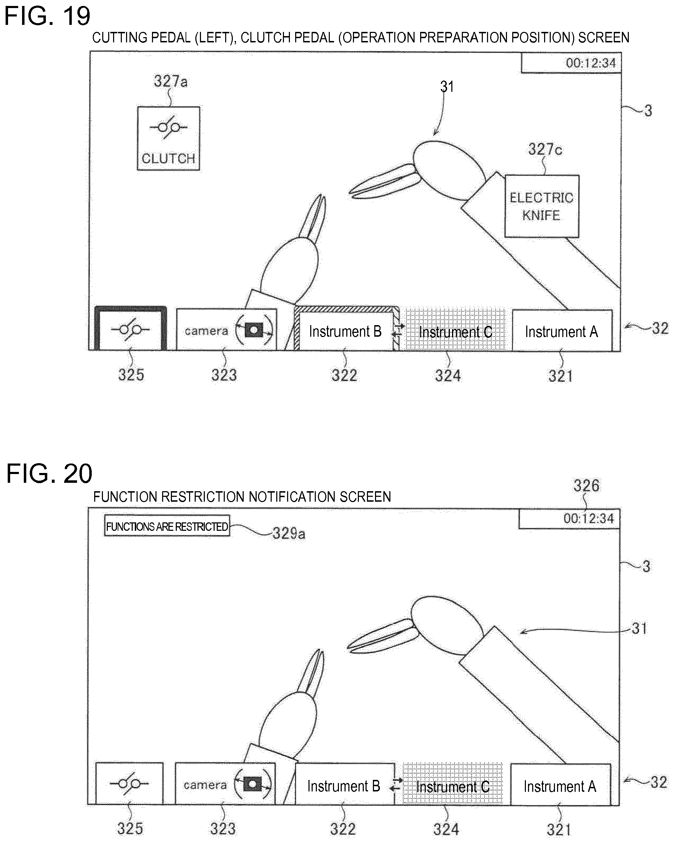

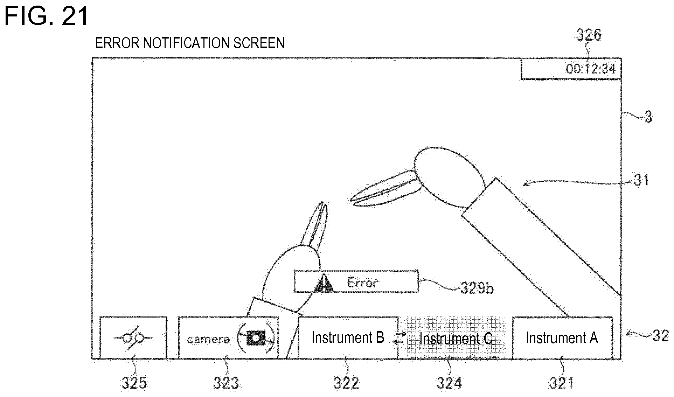

As illustrated in FIG. 7, the graphical user interface 32 displayed on the display device 3 includes the first area 321, the second area 322, the third area 323, a fourth area 324, and a fifth area 325. Additionally, the graphical user interface 32 includes a status area 326. Moreover, the graphical user interface 32 includes a pop-up area 327a (see FIG. 9), a pop-up area 327b (see FIG. 13), a pop-up area 327c (see FIG. 15), and a pop-up area 327d (see FIG. 17). Furthermore, the graphical user interface 32 includes a central area in which a level 328 (see FIG. 14) for the endoscope 201d is displayed. The graphical user interface 32 also includes an error notification area 329a (see FIG. 20) and an error notification area 329b (see FIG. 21).

The first area 321, second area 322, third area 323, fourth area 324, and fifth area 325 of the graphical user interface 32 are arranged side by side in a lower end region of the display device 3. The first area 321, second area 322, third area 323, fourth area 324, and fifth area 325 of the graphical user interface 32 may be arranged side by side in an upper end region of the display device 3. The pieces of information on the surgical instrument 201a, surgical instrument 201b, and endoscope 201d are displayed on the upper side or the lower side of the display device 3. Unlike the case where the pieces of information are displayed in the center region of the display device 3 other than the lower and upper end regions, it is possible to prevent the difficulty in viewing of the image 31 captured by the endoscope 201d caused by the first area 321, second area 322, third area 323, fourth area 324, and fifth area 325.

The pop-up areas 327a and 327b of the graphical user interface 32 are arranged on the left side in the right-left direction and near the center in the up-down direction of the display device 3. The pop-up areas 327c and 327d of the graphical user interface 32 are arranged on the right side in the right-left direction and near the center in the up-down direction of the display device 3. The status area 326 and the error notification area 329a of the graphical user interface 32 are arranged in the upper end region of the display device 3. The error notification area 329b is arranged above the second area 322 of the graphical user interface 32.

The first area 321 displays the type of the surgical instrument 201a being operated by the right-hand operation handle 1a. The second area 322 displays the type of the surgical instrument 201b being operated by the left-hand operation handle 1b. The third area 323 displays the information on the endoscope 201d. Thus, the operator O can easily grasp the types of the surgical instrument 201a being operated by the operation handle 1a and surgical instrument 201b being operated by the operation handle 1b. Additionally, the operator O can easily grasp the state of the endoscope 201d based on the information representing the endoscope 201d.

The fourth area 324 is arranged between the first area 321 and the second area 322. The fourth area 324 displays the information on the replacement surgical instrument 201c. Specifically, the fourth area 324 displays the type of the replacement surgical instrument 201c. Thus, the operator O can easily grasp, on the screen of the display device 3, the information on the replacement surgical instrument 201c, which is to be replaced with the surgical instrument 201a or the surgical instrument 201b and operated by the operation handle 1a or the operation handle 1b. The information in the fourth area 324 is displayed with paler color than the information in the first area 321 and second area 322. In other words, the information in the fourth area 324 is displayed to be more inconspicuous than the information in the first area 321 and second area 322.

The fifth area 325 is arranged to the left of the second area 322. The fifth area 325 displays the information on the clutch function. Specifically, the fifth area 325 displays the operation state of the clutch pedal 23. Thus, the operator O can easily grasp on the screen of the display device 3 the state of the clutch function of the clutch pedal 23, which is used when temporarily disconnecting the control-related connections between the manipulators 201 and the operation handles 1 and stopping the operations of the surgical instruments 201a and 201b.

The status area 326 displays the status of the surgical system 400. For example, the status area 326 displays duration of surgery procedure. The status area 326 also displays brightness adjustment information and contrast adjustment information of the display device 3.

The pop-up areas 327a to 327d are arranged in positions different from those of the first area 321, second area 322, and third area 323. The pop-up areas 327a to 327d display information, based on a fact that at least one of the sensors 26 detects setting of the foot of the operator O at the operation preparation position of the corresponding foot pedal 20. Thus, when the operator O sets the foot at the operation preparation position of the foot pedal 20, information on the foot pedal 20 is displayed in not only the corresponding area but in a different position as well. This allows the operator O to easily recognize setting of the foot pedal 20 to be operated at the operation preparation position.

As illustrated in FIG. 9, the pop-up area 327a displays the information on the clutch pedal 23 when the foot of the operator O is set at the operation preparation position of the clutch pedal 23. The pop-up area 327a displays information for a predetermined time (e.g., three seconds). Specifically, the display of the information in the pop-up area 327a ends when the foot of the operator O is moved from the operation preparation position of the clutch pedal 23, when the clutch pedal 23 is operated, or after a lapse of the predetermined time.

As illustrated in FIG. 13, the pop-up area 327b displays information on the camera pedal 24 when the foot of the operator O is set at the operation preparation position of the camera pedal 24. The pop-up area 327b displays information for a predetermined time (e.g., three seconds). Specifically, the display of the information in the pop-up area 327b ends when the foot of the operator O is moved from the operation preparation position of the camera pedal 24, when the camera pedal 24 is operated, or after a lapse of the predetermined time.

As illustrated in FIG. 15, the pop-up area 327c displays information on the cutting pedals 22 when the foot of the operator O is set at the operation preparation position of the cutting pedal 22a or 22b. The pop-up area 327c displays information for a predetermined time (e.g., three seconds). Specifically, the display of the information in the pop-up area 327c ends when the foot of the operator O is moved from the operation preparation position of the cutting pedal 22a or 22b, when the cutting pedal 22a or 22b is operated, or after a lapse of the predetermined time.

As illustrated in FIG. 17, the pop-up area 327d displays information on the coagulation pedals 21 when the foot of the operator O is set at the operation preparation position of the coagulation pedal 21a or 21b. The pop-up area 327d displays information for a predetermined time (e.g., three seconds). Specifically, the display of the information in the pop-up area 327d ends when the foot of the operator O is moved from the operation preparation position of the coagulation pedal 21a or 21b, when the coagulation pedal 21a or 21b is operated, or after a lapse of the predetermined time.

As illustrated in FIG. 14, the level 328 for the endoscope 201d is displayed on the display device 3 when the camera pedal 24 is operated. Thus, the operator O can easily change the orientation of the endoscope 201d through operation referring to the displayed level 328.

In an embodiment, the control apparatus 6 is configured to change the display form of a corresponding area out of the first area 321, the second area 322, and the third area 323, based on a fact that at least one of the sensors 26 detects setting of the foot of the operator O at the operation preparation position of at least one foot pedal 20 out of the right-side foot pedals (the coagulation pedal 21b and the cutting pedal 22b), the left-side foot pedals (the coagulation pedal 21a and the cutting pedal 22a), and the camera pedal 24. Thus, when the foot of the operator O is set at the operation preparation position of the foot pedal 20, the graphical user interface 32 corresponding to the foot pedal 20 is displayed on the display device 3 with the display form of the area of the graphical user interface 32 changed. Consequently, the operator O does not need to check with the eyes that actually the foot is located on which foot pedal 20. Thus, the operator O can confirm the foot pedal 20 to be operated while keeping eyes on the display device 3.

The changing of the display form of an area includes at least one of display color change, inverted display, display change of an area frame line, and zoom/zoom-out of the display area. When the operator O sets the foot at the operation preparation position of the foot pedal 20, the display color change, inverted display, or display change of the area frame line occurs in the corresponding area. Consequently, the operator O can easily recognize the changing of the display form and can easily confirm the foot pedal 20 to be operated.

The control apparatus 6 is configured to display the information with the first color in a corresponding area out of the first area 321 and the second area 322, based on a fact that at least one of the sensors 26 detects setting of the foot of the operator O at the operation preparation position of the coagulation pedal 21a or 21b. Additionally, the control apparatus 6 is configured to display the information with the second color in a corresponding area out of the first area 321 and the second area 322, based on a fact that at least one of the sensors 26 detects setting of the foot of the operator O at the operation preparation position of the cutting pedal 22a or 22b. It is possible to display the information in the corresponding areas with different colors between the case of operating the coagulation pedal 21a or 21b and the case of operating the cutting pedal 22a or 22b. Consequently, the operator O can easily recognize whether the foot pedal 20 to be operated is the coagulation pedal 21a or 21b or the cutting pedal 22a or 22b while keeping eyes on the display device 3.

The control apparatus 6 is configured, when the foot pedal 20 is operated, to display, in the corresponding area, the information in a display form different from a display form in a case where the foot of the operator O is set at the operation preparation position of the foot pedal 20. That is, a display form in the state where the foot of the operator O is set at the operation preparation position is different from a display form in the state where the foot of the operator O is operated. Consequently, the operator O can easily recognize whether the foot pedal 20 is in an operation preparation state or in an operated state.

The areas of the graphical user interface 32 are configured to display the operation states of the corresponding foot pedals 20. Thus, the operator O can easily grasp the operation states of the foot pedals 20.

Specifically, in addition to the information on the surgical instrument 201a, the first area 321 displays the information on the right-side foot pedals (the coagulation pedal 21b and the cutting pedal 22b). For example, when the foot of the operator O is set at the operation preparation position of the coagulation pedal 21b or the cutting pedal 22b, the frame line of the first area 321 is displayed with the color of the corresponding foot pedal 20 (the coagulation pedal 21b or the cutting pedal 22b). Thus, the operator O can easily grasp the execution state of the function of the surgical instrument 201a operated by the right-side foot pedals (the coagulation pedal 21b and the cutting pedal 22b) through the display of the first area 321. When the coagulation pedal 21b or the cutting pedal 22b is operated, the first area 321 displays the information on the coagulation pedal 21b or the cutting pedal 22b such that the display is distinguished from that in the case where the foot of the operator O is set at the operation preparation position. For example, when the coagulation pedal 21b or the cutting pedal 22b is operated, the entirety of the first area 321 is displayed with the color of the corresponding foot pedal 20 (the coagulation pedal 21b or the cutting pedal 22b). Thus, the operator O can easily grasp the operation state of the coagulation pedal 21b or the cutting pedal 22b through the display of the first area 321.

In other words, when the coagulation pedal 21b is operated, the first area 321 displays the information on the coagulation pedal 21b. When the cutting pedal 22b is operated, the first area 321 displays the information on the cutting pedal 22b. Consequently, the operator O can easily grasp the execution states of the coagulation function and the cutting function by the surgical instrument 201a that is operated by the right hand-operation handle 1a through the display of the first area 321.

In addition to the information on the surgical instrument 201b, the second area 322 displays the information on the left-side foot pedals (the coagulation pedal 21a and the cutting pedal 22a). For example, when the foot of the operator O is set at the operation preparation position of the coagulation pedal 21a or the cutting pedal 22a, the frame line of the second area 322 is displayed with the color of the corresponding foot pedal 20 (the coagulation pedal 21a or the cutting pedal 22a). Thus, the operator O can easily grasp the execution state of the function of the surgical instrument 201b operated by the left-side foot pedals (the coagulation pedal 21a and the cutting pedal 22a) through the display of the second area 322. When the coagulation pedal 21a or the cutting pedal 22a is operated, the second area 322 displays the information on the coagulation pedal 21a or the cutting pedal 22a such that the display is distinguished from that in the case where the foot of the operator O is set at the operation preparation position. For example, when the coagulation pedal 21a or the cutting pedal 22a is operated, the entirety of the second area 322 is displayed with the color of the corresponding foot pedal 20 (the coagulation pedal 21a or the cutting pedal 22a). Thus, the operator O can easily grasp the operation state of the coagulation pedal 21a or the cutting pedal 22a through the display of the second area 322.

In other words, when the coagulation pedal 21a is operated, the second area 322 displays the information on the coagulation pedal 21a. When the cutting pedal 22a is operated, the second area 322 displays the information on the cutting pedal 22a. Consequently, the operator O can easily grasp the execution states of the coagulation function and the cutting function by the surgical instrument 201b that is operated by the left hand-operation handle 1b through the display of the second area 322.

In addition to the information on the endoscope 201d, the third area 323 displays the information on the camera pedal 24. For example, when the foot of the operator O is set at the operation preparation position of the camera pedal 24, the frame line of the third area 323 is emphasized. Thus, the operator O can easily grasp the execution state of the function of the endoscope 201d operated by the camera pedal 24 through the display of the third area 323. When the camera pedal 24 is operated, the third area 323 displays the information on the camera pedal 24 such that the display is distinguished from that in the case where the foot of the operator O is set at the operation preparation position. For example, when the camera pedal 24 is operated, the background color and the text color of the third area 323 are inverted. Thus, the operator O can easily grasp the operation state of the camera pedal 24 through the display of the third area 323.

Next, with reference to FIGS. 8 to 21, an example of the graphical user interface 32 displayed based on the operation of the foot pedals 20 by the operator O.

As illustrated in FIG. 8, when the switch pedal 25 is operated, the surgical instrument operated by the left hand-operation handle 1b is switched from the surgical instrument 201b to the surgical instrument 201c. In this case, the background colors and the text colors of the second area 322 and the fourth area 324 are inverted. Thus, the operator O can easily recognize the switching of the surgical instruments.

As illustrated in FIG. 9, when the foot of the operator O is set at the operation preparation position of the clutch pedal 23, the frame line of the fifth area 325 is emphasized. The information on the clutch pedal 23 is popped up and displayed in the pop-up area 327a. Even when the foot of the operator O continues to be set at the operation preparation position of the clutch pedal 23, the display of the pop-up area 327a ends after a lapse of the predetermined time. In this case, the emphasized display of the frame line of the fifth area 325 continues.

As illustrated in FIG. 10, when the clutch pedal 23 is operated, the control-related connections between the operation handles 1 and the manipulators 201 are temporarily disconnected. In this case, the background color and the text color of the fifth area 325 displaying the information on the clutch pedal 23 are inverted. Additionally, the background color and the text color of the first area 321 displaying the information on the surgical instrument 201a in which the control-related connection is temporarily disconnected are inverted. Moreover, the background color and the text color of the second area 322 displaying the information on the surgical instrument 201b in which the control-related connection is temporarily disconnected are inverted. When the foot of the operator O goes back to the operation preparation position after the operation, the frame line of the fifth area 325 is emphasized as illustrated in FIG. 9.

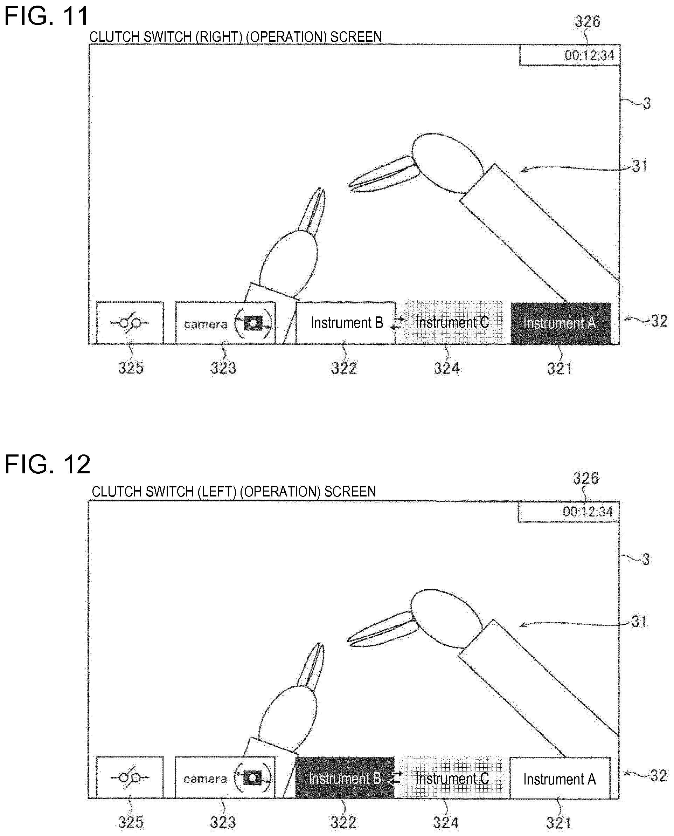

As illustrated in FIG. 11, when the clutch switch 11 of the right hand-operation handle 1a is operated, the control-related connection between the operation handle 1a and the manipulator 201 provided with the surgical instrument 201a is temporarily disconnected. In this case, the background color and text color of the first area 321 displaying the information on the surgical instrument 201a in which the control-related connection is temporarily disconnected are inverted.

As illustrated in FIG. 12, when the clutch switch 11 of the left hand-operation handle 1b is operated, the control-related connection between the operation handle 1b and the manipulator 201 provided with the surgical instrument 201b is temporarily disconnected. In this case, the background color and the text color of the second area 322 displaying the information on the surgical instrument 201b in which the control-related connection is temporarily disconnected are inverted.

As illustrated in FIG. 13, when the foot of the operator O is set at the operation preparation position of the camera pedal 24, the frame line of the third area 323 is emphasized. Additionally, the information on the camera pedal 24 is popped up and displayed in the pop-up area 327b. Even when the foot of the operator O continues to be set at the operation preparation position of the camera pedal 24, the display of the pop-up area 327b ends after a lapse of the predetermined time. In this case, the emphasized display of the frame line of the third area 323 continues.

As illustrated in FIG. 14, when the camera pedal 24 is operated, the position and orientation of the endoscope 201d can be controlled by the operation handles 1a and 1b. In this process, the background color and the text color of the third area 323 displaying the information on the camera pedal 24 are inverted. Additionally, the level 328 for the endoscope 201d is displayed in the central area.

As illustrated in FIG. 15, when the foot of the operator O is set at the operation preparation position of the cutting pedal 22b corresponding to the surgical instrument 201a being operated by the right hand, the frame line of the first area 321 is emphasized. Specifically, more than left half of the frame line is emphasized by the second color of the cutting pedal 22b. The rest of the frame line is emphasized by the first color of the coagulation pedal 21b. Thus, comparing with a case where the frame line is emphasized by only the second color, the operator O can more effectively recognize setting of the foot at the operation preparation position of the cutting pedal 22b out of the cutting pedal 22b and the coagulation pedal 21b. Additionally, the information on the cutting pedal 22b is popped up and displayed in the pop-up area 327c. Even when the foot of the operator O continues to be set at the operation preparation position of the cutting pedal 22b, the display of the pop-up area 327c ends after a lapse of the predetermined time. In this case, the emphasized display of the frame line of the first area 321 continues.

Likewise, the frame line of the second area 322 is emphasized also when the foot of the operator O is set at the operation preparation position of the cutting pedal 22a corresponding to the surgical instrument 201b being operated by the left hand.

As illustrated in FIG. 16, when the cutting pedal 22b is operated, predetermined voltage is applied to the surgical instrument 201a to enable cutting of the surgery site. In this process, the background of the first area 321 displaying the information on the cutting pedal 22b is displayed with the second color of the cutting pedal 22b.

Likewise, also when the cutting pedal 22a is operated, the background of the second area 322 displaying the information on the cutting pedal 22a is displayed with the second color of the cutting pedal 22a.

As illustrated in FIG. 17, when the foot of the operator O is set at the operation preparation position of the coagulation pedal 21b corresponding to the surgical instrument 201a being operated by the right hand, the frame line of the first area 321 is emphasized. Specifically, more than right half of the frame line is emphasized by the first color of the coagulation pedal 21b. The rest of the frame line is emphasized by the second color of the cutting pedal 22b. Thus, comparing with a case where the frame line is emphasized by only the first color, the operator O can more effectively recognize setting of the foot at the operation preparation position of the coagulation pedal 21b out of the cutting pedal 22b and the coagulation pedal 21b. Additionally, the information on the coagulation pedal 21b is popped up and displayed in the pop-up area 327d. Even when the foot of the operator O continues to be set at the operation preparation position of the coagulation pedal 21b, the display of the pop-up area 327d ends after a lapse of the predetermined time. In this case, the emphasized display of the frame line of the first area 321 continues.

Likewise, the frame line of the second area 322 is emphasized also when the foot of the operator O is set at the operation preparation position of the coagulation pedal 21a corresponding to the surgical instrument 201b being operated by the left hand.

As illustrated in FIG. 18, when the coagulation pedal 21b is operated, predetermined voltage is applied to the surgical instrument 201a to enable cutting of the surgery site. In this process, the background of the first area 321 displaying the information on the coagulation pedal 21b is displayed with the first color of the coagulation pedal 21b.

Likewise, also when the coagulation pedal 21a is operated, the background of the second area 322 displaying the information on the coagulation pedal 21a is displayed with the first color of the coagulation pedal 21a.

As illustrated in FIG. 19, when the foot of the operator O is set at the operation preparation position of the cutting pedal 22a corresponding to the surgical instrument 201b being operated by the left hand and the other foot of the operator O is set at the operation preparation position of the clutch pedal 23, the frame line of the second area 322 is emphasized. Specifically, more than left half of the frame line is emphasized by the second color of the cutting pedal 22a. The rest of the frame line is emphasized by the first color of the coagulation pedal 21a. Additionally, the information on the cutting pedal 22a is popped up and displayed in the pop-up area 327c. Moreover, the frame line of the fifth area 325 is emphasized. The information on the clutch pedal 23 is popped up and displayed in the pop-up area 327a.

As illustrated in FIG. 20, when the functions are restricted, a notification to indicate that the functions are restricted is displayed in the error notification area 329a. When the cutting pedals 22 and the coagulation pedals 21 are operated while a surgical instrument in which the functions of cutting and coagulating are not set, a notification to indicate that unfunctional operation is tried to be executed is popped up and displayed on the upper side of the area corresponding to the surgical instrument. In this case, no voltage is applied to the surgical instrument even if the cutting pedals 22 and the coagulation pedals 21 are operated.

As illustrated in FIG. 21, when an error is notified, the error notification is displayed in the error notification area 329b.

### (Modifications)

The one or more embodiments disclosed above should be construed as an example in all aspects and should not limit the invention. The scope of the invention is indicated by the scope of claim instead of by the above-described one or more embodiments.

For example, the foot pedals on the operation pedal section include the coagulation pedals and the cutting pedals in the example illustrated in the above-described one or more embodiments, but the invention is not limited thereto. In an embodiment or a modification, the foot pedals on the operation pedal section may include a foot pedal that executes a function of medical equipment other than the coagulation pedals and the cutting pedals.

The sensor is the blockage type in the example illustrated in the above-described one or more embodiments, but the invention is not limited thereto. The sensor may be a transparent type or a reflection type. Otherwise, the sensor may detect the presence of the foot without using light. For example, the detection may be performed by using a soundwave or a coil.

## Claims

1. A surgical system (400), comprising:
manipulators (201) that respectively support an endoscope (201d), a first surgical instrument (201a), and a second surgical instrument (201b);
a remote control apparatus (100) that includes a display device (3) configured to display an image captured by the endoscope, a first operation handle (1a) to operate the first surgical instrument, a second operation handle (1b) to operate the second surgical instrument, and an operation pedal section (2) including foot pedals (20); and
a control apparatus (63) configured to control at least the display device, wherein
the operation pedal section includes contactless type sensors (26) configured to detect the presence of a foot of an operator coming closer to the respective foot pedals,
the foot pedals include a first foot pedal (21b, 22b) to execute a function of the first surgical instrument, a second foot pedal (21a, 22a) to execute a function of the second surgical instrument, and a third foot pedal (24) to execute a function of the endoscope,
the control apparatus is configured to display a graphical user interface (32), overlapped with the image captured by the endoscope, on the display device, the graphical user interface including a first area (321) that displays information on the first surgical instrument, a second area (322) that displays information on the second surgical instrument, and a third (323) area that displays information on the endoscope which are arranged therein, and
the control apparatus is further configured to change, when the foot of the operator coming closer to at least one of the first to third foot pedals is detected by at least one of the sensors, a display form of corresponding at least one of the first to third areas.

2. The surgical system according to claim 1, wherein
the graphical user interface includes the first area, the second area, and the third area in a lower end region or in an upper end region of the display device.

3. The surgical system according to claim 1 or 2, wherein
changing of the display form of the corresponding at least one area includes at least one of display color change, inverted display, and display change of a frame line of the corresponding at least one area.

4. The surgical system according to any one of claims 1 to 3, wherein
the control apparatus is configured to display, when the foot of the operator coming closer to at least one of the first to third foot pedals is detected by at least one of the sensors, information on the at least one foot pedal to which the foot of the operator is coming closer, the information being displayed in a position different from those of the first to third areas.

5. The surgical system according to any one of claims 1 to 4, wherein
the first area displays a type of the first surgical instrument,
the second area displays a type of the second surgical instrument, and
the third area displays information on the endoscope.

6. The surgical system according to claim 5, wherein
the first foot pedal includes a first coagulation pedal (21b) to coagulate a surgery site and a first cutting pedal (22b) to cut the surgery site,
the second foot pedal includes a second coagulation pedal (21a) to coagulate the surgery site and a second cutting pedal (22a) to cut the surgery site,
when the first coagulation pedal is operated, the first area displays information on the first coagulation pedal, different from that in the case where the foot of the operator is coming closer to the first coagulation pedal,
when the first cutting pedal is operated, the first area displays information on the first cutting pedal, different from that in the case where the foot of the operator is coming closer to the first cutting pedal,
when the second coagulation pedal is operated, the second area displays information on the second coagulation pedal, different from that in the case where the foot of the operator is coming closer to the second coagulation pedal, and
when the second cutting pedal is operated, the second area displays information on the second cutting pedal, different from that in the case where the foot of the operator is coming closer to the second cutting pedal.

7. The surgical system according to claim 6, wherein
the first coagulation pedal and the second coagulation pedal are in a first color,
the first cutting pedal and the second cutting pedal are in a second color different from the first color, and
the control apparatus is configured to display, when the foot of the operator coming closer to one of the first coagulation pedal and the second coagulation pedal is detected by at least one of the sensors, information with the first color in a corresponding one of the first area and the second area, and
the control apparatus is further configured to display, when the foot of the operator coming closer to one of the first cutting pedal and the second cutting pedal is detected by at least one of the sensors, information with the second color in a corresponding one of the first area and the second area.

8. The surgical system according to any one of claims 1 to 7, wherein
the control apparatus is configured to display, when one of the first to third foot pedals is operated, information in a corresponding one of the first to third areas, the information being displayed in a display form different from a display form in a case where the foot of the operator is coming closer to the foot pedal.

9. The surgical system according to any one of claims 1 to 8, wherein
one of the manipulators supports a replacement surgical instrument (201c),
the operation pedal section further includes a fourth foot pedal (25) to replace one of the first surgical instrument to be operated by the first operation handle and the second surgical instrument to be operated by the second operation handle with the replacement surgical instrument, and
the graphical user interface includes a fourth area (324) that is arranged between the first area and the second area and displays information on the replacement surgical instrument.

10. The surgical system according to any one of claims 1 to 9, wherein
the operation pedal section further includes a fifth foot pedal (23) to execute a clutch function, and
the graphical user interface includes a fifth area (325) that displays information on the clutch function.

11. The surgical system according to any one of claims 1 to 10, wherein
the control apparatus is configured to display, when the third foot pedal is operated, the graphical user interface including display of a level (328) for the endoscope, overlapped with the image captured by the endoscope.

12. The surgical system according to any one of claims 1 to 11 , wherein
each of the first to third areas of the graphical user interface displays an operation state of a corresponding foot pedal.

13. A method of displaying information in a surgical system (400),
the surgical system including: manipulators (201) that respectively support an endoscope (201d), a first surgical instrument (201a), and a second surgical instrument (201b); a remote control apparatus (100) that includes a display device (3) configured to display an image captured by the endoscope, a first operation handle (1a) to operate the first surgical instrument, and a second operation handle (1b) to operate the second surgical instrument; an operation pedal section including foot pedals; and a control apparatus configured to control at least the display device,
the operation pedal section includes contactless type sensors (26) that detect the presence of a foot of an operator coming closer to the respective foot pedals,
the foot pedals include a first foot pedal (21b, 22b) to execute a function of the first surgical instrument, a second foot pedal (21a, 22a) to execute a function of the second surgical instrument, and a third foot pedal (24) to execute a function of the endoscope, the method comprising:
displaying a graphical user interface (32) including a first area (321) that displays information on the first surgical instrument, a second area (322) that displays information on the second surgical instrument, and a third area (323) that displays information on the endoscope; and
changing, when the foot of the operator coming closer to at least one of the first to third foot pedals is detected by at least one of the sensors, a display form of corresponding at least one of the first to third areas.

14. The method according to claim 13, wherein
changing of the display form of the corresponding at least one area includes at least one of display color change, inverted display, and display change of a frame line of the corresponding at least one area.

15. The method according to claim 13, wherein
displaying, when the third foot pedal is operated, the graphical user interface including display of a level (328) for the endoscope, with being overwrapped with the image captured by the endoscope.

## Patentansprüche

1. Chirurgisches System (400), aufweisend:
Manipulatoren (201), die jeweils ein Endoskop (201d), ein erstes chirurgisches Instrument (201a) und ein zweites chirurgisches Instrument (201b) tragen;
eine Fernsteuerungseinrichtung (100), die eine Anzeigevorrichtung (3), die dafür konfiguriert ist, ein mittels des Endoskops aufgenommenes Bild anzuzeigen, einen ersten Bedienungsgriff (1a), um das erste chirurgische Instrument zu bedienen, einen zweiten Bedienungsgriff (1b), um das zweite chirurgische Instrument zu bedienen, und eine Fußpedale (20) enthaltende Bedienungspedalsektion (2) umfasst; und
eine Steuerungseinrichtung (63), die dafür konfiguriert ist, zumindest die Anzeigevorrichtung zu steuern, wobei
die Bedienungspedalsektion Sensoren (26) vom kontaktlosen Typ enthält, die dafür konfiguriert sind, die Präsenz eines sich den jeweiligen Fußpedalen nähernden Fußes eines Bedieners zu detektieren,
die Fußpedale ein erstes Fußpedal (21b, 22b), um eine Funktion des ersten chirurgischen Instruments auszuführen, ein zweites Fußpedal (21a, 22a), um eine Funktion des zweiten chirurgischen Instruments auszuführen, und ein drittes Fußpedal (24), um eine Funktion des Endoskops auszuführen, umfassen,
die Steuerungseinrichtung dafür konfiguriert ist, eine dem mittels des Endoskops aufgenommenen Bild überlagerte graphische Nutzerschnittstelle (32) auf der Anzeigevorrichtung anzuzeigen, wobei die graphische Nutzerschnittstelle (32) einen ersten Bereich (321), der Informationen über das erste chirurgische Instrument anzeigt, einen zweiten Bereich (322), der Informationen über das zweite chirurgische Instrument anzeigt, und einen dritten Bereich (323), der Informationen über das Endoskop anzeigt, enthält, die darin angeordnet sind, und
die Steuerungseinrichtung ferner dafür konfiguriert ist, wenn durch zumindest einen der Sensoren der sich zumindest einem der ersten bis dritten Fußpedale nähernde Fuß des Bedieners detektiert wird, eine Anzeigeform eines entsprechenden, zumindest einen der ersten bis dritten Bereiche zu ändern.

2. Chirurgisches System nach Anspruch 1, wobei
die graphische Nutzerschnittstelle den ersten Bereich, den zweiten Bereich und den dritten Bereich in einem unteren Endbereich oder in einem oberen Endbereich der Anzeigevorrichtung enthält.

3. Chirurgisches System nach Anspruch 1 oder 2, wobei
ein Ändern der Anzeigeform des entsprechenden, zumindest einen Bereichs eine Änderung der Anzeigefarbe, eine invertierte Anzeige und/oder eine Anzeigeänderung einer Rahmenlinie des entsprechenden, zumindest einen Bereichs einschließt.

4. Chirurgisches System nach einem der Ansprüche 1 bis 3, wobei
die Steuerungseinrichtung dafür konfiguriert ist, wenn durch zumindest einen der Sensoren der sich zumindest einem der ersten bis dritten Fußpedale nähernde Fuß des Bedieners detektiert wird, Informationen über das zumindest eine Fußpedal anzuzeigen, dem sich der Fuß des Bedieners nähert, wobei die Informationen an einer von jenen der ersten bis dritten Bereiche verschiedenen Position angezeigt werden.

5. Chirurgisches System nach einem der Ansprüche 1 bis 4, wobei
der erste Bereich einen Typ des ersten chirurgischen Instruments anzeigt,
der zweite Bereich einen Typ des zweiten chirurgischen Instruments anzeigt und
der dritte Bereich Informationen über das Endoskop anzeigt.

6. Chirurgisches System nach Anspruch 5, wobei
das erste Fußpedal ein erstes Koagulationspedal (21b), um eine Operationsstelle zu koagulieren, und ein erstes Schneidpedal (22b), um die Operationsstelle zu schneiden, umfasst,
das zweite Fußpedal ein zweites Koagulationspedal (21a), um die Operationsstelle zu koagulieren, und ein zweites Schneidpedal (22a), um die Operationsstelle zu schneiden, umfasst,
wenn das erste Koagulationspedal betätigt wird, der erste Bereich Informationen über das erste Koagulationspedal anzeigt, die von jenen in dem Fall, in dem sich der Fuß des Bedieners dem ersten Koagulationspedal nähert, verschieden sind,
wenn das erste Schneidpedal betätigt wird, der erste Bereich Informationen über das erste Schneidpedal anzeigt, die von jenen in dem Fall, in dem sich der Fuß des Bedieners dem ersten Schneidpedal nähert, verschieden sind,
wenn das zweite Koagulationspedal betätigt wird, der zweite Bereich Informationen über das zweite Koagulationspedal anzeigt, die von jenen in dem Fall, in dem sich der Fuß des Bedieners dem zweiten Koagulationspedal nähert, verschieden sind, und,
wenn das zweite Schneidpedal betätigt wird, der zweite Bereich Informationen über das zweite Schneidpedal anzeigt, die von jenen in dem Fall, in dem sich der Fuß des Bedieners dem zweiten Schneidpedal nähert, verschieden sind.

7. Chirurgisches System nach Anspruch 6, wobei
das erste Koagulationspedal und das zweite Koagulationspedal in einer ersten Farbe vorliegen,
das erste Schneidpedal und das zweite Schneidpedal in einer von der ersten Farbe verschiedenen zweiten Farbe vorliegen,
die Steuerungseinrichtung dafür konfiguriert ist, wenn durch zumindest einen der Sensoren der sich einem des ersten Koagulationspedals und des zweiten Koagulationspedals nähernde Fuß des Bedieners detektiert wird, Informationen mit der ersten Farbe in einem entsprechenden des ersten Bereichs und des zweiten Bereichs anzuzeigen, und
die Steuerungseinrichtung ferner dafür konfiguriert ist, wenn durch zumindest einen der Sensoren der sich einem des ersten Schneidpedals und des zweiten Schneidpedals nähernde Fuß des Bedieners detektiert wird, Informationen mit der zweiten Farbe in einem entsprechenden des ersten Bereichs und des zweiten Bereichs anzuzeigen.

8. Chirurgisches System nach einem der Ansprüche 1 bis 7, wobei
die Steuerungseinrichtung dafür konfiguriert ist, wenn eines der ersten bis dritten Fußpedale betätigt wird, Informationen in einem entsprechenden der ersten bis dritten Bereiche anzuzeigen, wobei die Informationen in einer Anzeigeform angezeigt werden, die von einer Anzeigeform in einem Fall, in dem sich der Fuß des Bedieners dem Fußpedal nähert, verschieden ist.

9. Chirurgisches System nach einem der Ansprüche 1 bis 8, wobei
einer der Manipulatoren ein chirurgisches Austauschinstrument (201c) trägt,
die Bedienungspedalsektion ferner ein viertes Fußpedal (25) aufweist, um eines des ersten chirurgischen Instruments, das mittels des ersten Bedienungsgriffs zu bedienen ist, und des zweiten chirurgischen Instruments, das mittels des zweiten Bedienungsgriffs zu bedienen ist, durch das chirurgische Austauschinstrument zu ersetzen, und
die graphische Nutzerschnittstelle einen vierten Bereich (324) enthält, der zwischen dem ersten Bereich und dem zweiten Bereich angeordnet ist und Informationen über das chirurgische Austauschinstrument anzeigt.

10. Chirurgisches System nach einem der Ansprüche 1 bis 9, wobei
die Bedienungspedalsektion ferner ein fünftes Fußpedal (23) enthält, um eine Kupplungsfunktion auszuführen, und
die graphische Nutzerschnittstelle einen fünften Bereich (325) enthält, der Informationen über die Kupplungsfunktion anzeigt.

11. Chirurgisches System nach einem der Ansprüche 1 bis 10, wobei
die Steuerungseinrichtung dafür konfiguriert ist, wenn das dritte Fußpedal betätigt wird, die graphische Nutzerschnittstelle, die eine Anzeige eines Niveaus (328) für das Endoskop enthält, mit dem mittels des Endoskops aufgenommenen Bild überlagert anzuzeigen.

12. Chirurgisches System nach einem der Ansprüche 1 bis 11, wobei
jeder der ersten bis dritten Bereiche der graphischen Nutzerschnittstelle einen Betätigungszustand eines entsprechenden Fußpedals anzeigt.

13. Verfahren zum Anzeigen von Informationen in einem chirurgischen System (400),
wobei das chirurgische System umfasst: Manipulatoren (201), die jeweils ein Endoskop (201d), ein erstes chirurgisches Instrument (201a) und ein zweites chirurgisches Instrument (201b) tragen; eine Fernsteuerungseinrichtung (100), die eine Anzeigevorrichtung (3), die dafür konfiguriert ist, ein mittels des Endoskops aufgenommenes Bild anzuzeigen, einen ersten Bedienungsgriff (1a), um das erste chirurgische Instrument zu bedienen, und einen zweiten Bedienungsgriff (1b), um das zweite chirurgische Instrument zu bedienen, umfasst; eine Fußpedale enthaltende Bedienungspedalsektion; und eine Steuerungseinrichtung, die dafür konfiguriert ist, zumindest die Anzeigevorrichtung zu steuern,
die Bedienungspedalsektion Sensoren (26) vom kontaktlosen Typ enthält, die die Präsenz eines sich den jeweiligen Fußpedalen nähernden Fußes eines Bedieners detektieren,
die Fußpedale ein erstes Fußpedal (21b, 22b), um eine Funktion des ersten chirurgischen Instruments auszuführen, ein zweites Fußpedal (21a, 22a), um eine Funktion des zweiten chirurgischen Instruments auszuführen, und ein drittes Fußpedal (24), um eine Funktion des Endoskops auszuführen, umfassen, wobei das Verfahren aufweist:
ein Anzeigen einer graphischen Nutzerschnittstelle (32), die einen ersten Bereich (321), der Informationen über das erste chirurgische Instrument anzeigt, einen zweiten Bereich (322), der Informationen über das zweite chirurgische Instrument anzeigt, und einen dritten Bereich (323), der Informationen über das Endoskop anzeigt, enthält; und
ein Ändern, wenn durch zumindest einen der Sensoren der sich zumindest einem der ersten bis dritten Fußpedale nähernde Fuß des Bedieners detektiert wird, einer Anzeigeform eines entsprechenden, zumindest einen der ersten bis dritten Bereiche.

14. Verfahren nach Anspruch 13, wobei
ein Ändern der Anzeigeform des entsprechenden, zumindest einen Bereichs eine Änderung der Anzeigefarbe, eine invertierte Anzeige und/oder eine Anzeigeänderung einer Rahmenlinie des entsprechenden, zumindest einen Bereichs einschließt.

15. Verfahren nach Anspruch 13, wobei
ein Anzeigen, wenn das dritte Fußpedal betätigt wird, der graphischen Nutzerschnittstelle eine Anzeige eines Niveaus (328) für das Endoskop einschließt, wobei sie dem mittels des Endoskops aufgenommenen Bild überlagert wird.

## Revendications

1. Système chirurgical (400), comprenant :
des manipulateurs (201) qui supportent respectivement un endoscope (201d), un premier instrument chirurgical (201a), et un deuxième instrument chirurgical (201b) ;
un appareil de commande distant (100) qui inclut un dispositif d'affichage (3) configuré pour afficher une image capturée par l'endoscope, une première manette d'actionnement (1a) pour actionner le premier instrument chirurgical, une deuxième manette d'actionnement (1b) pour actionner le deuxième instrument chirurgical, et une section de pédales d'actionnement (2) incluant des pédales de pied (20) ; et
un appareil de commande (63) configuré pour commander au moins le dispositif d'affichage, dans lequel
la section de pédales d'actionnement inclut des capteurs de type sans contact (26) configurés pour détecter la présence d'un pied d'un opérateur se rapprochant des pédales de pied respectives,
les pédales de pied incluent une première pédale de pied (21b, 22b) pour exécuter une fonction du premier instrument chirurgical, une deuxième pédale de pied (21a, 22a) pour exécuter une fonction du deuxième instrument chirurgical, et une troisième pédale de pied (24) pour exécuter une fonction de l'endoscope,
l'appareil de commande est configuré pour afficher sur le dispositif d'affichage une surface d'utilisateur graphique (32) empiétant sur l'image capturée par l'endoscope, l'interface d'utilisateur graphique incluant une première zone (321) qui affiche des informations sur le premier instrument chirurgical, une deuxième zone (322) qui affiche des informations sur le deuxième instrument chirurgical, et une troisième (323) zone qui affiche des informations sur l'endoscope, qui sont agencées dans celle-ci, et
l'appareil de commande est en outre configuré pour changer, quand le pied de l'opérateur se rapprochant de l'une au moins des première à troisième pédales de pied est détecté par l'un au moins des capteurs, une forme d'affichage de l'une au moins des première à troisième zones correspondante.

2. Système chirurgical selon a revendication 1, dans lequel
l'interface d'utilisateur graphique inclut la première zone, la deuxième zone, et la troisième zone dans une région d'extrémité inférieure ou dans une région d'extrémité supérieure du dispositif d'affichage.

3. Système chirurgical selon la revendication 1 ou 2, dans lequel
le changement de la forme d'affichage de l'une au moins des zones correspondante inclut l'un au moins d'un changement de couleur d'affichage, d'un affichage inversé, et d'un changement d'affichage d'une ligne de cadre de l'une au moins des zones correspondante.

4. Système chirurgical selon l'une quelconque des revendications 1 à 3, dans lequel
l'appareil de commande est configuré pour afficher, quand le pied de l'opérateur se rapprochant de l'une au moins des première à troisième pédales de pied est détecté par l'un au moins des capteurs, des informations sur la au moins une pédale de pied dont le pied de l'opérateur se rapproche, les informations étant affichées dans une position différente de celles des première à troisième zones.

5. Système chirurgical selon l'une quelconque des revendications 1 à 4, dans lequel
la première zone affiche un type du premier instrument chirurgical,
la deuxième zone affiche un type du deuxième instrument chirurgical, et
la troisième zone affiche des informations sur l'endoscope.

6. Système chirurgical selon la revendication 5, dans lequel
la première pédale de pied inclut une première pédale de coagulation (21b) pour coaguler un site chirurgical et une première pédale de coupe (22b) pour couper le site chirurgical,
la deuxième pédale de pied inclut une deuxième pédale de coagulation (21a) pour coaguler le site chirurgical et une deuxième pédale de coupe (22a) pour couper le site chirurgical,
quand la première pédale de coagulation est actionnée, la première zone affiche des informations sur la première pédale de coagulation, différentes de celles dans le cas où le pied de l'opérateur se rapproche de la première pédale de coagulation,
quand la première pédale de coupe est actionnée, la première zone affiche des informations sur la première pédale de coupe, différentes de celles dans le cas où le pied de l'opérateur se rapproche de la première pédale de coupe,
quand la deuxième pédale de coagulation est actionnée, la deuxième zone affiche des informations sur la deuxième pédale de coagulation, différentes de celles dans le cas où le pied de l'opérateur se rapproche de la deuxième pédale de coagulation, et
quand la deuxième pédale de coupe est actionnée, la deuxième zone affiche des informations sur la deuxième pédale de coupe, différentes de celles dans le cas où le pied de l'opérateur se rapproche de la deuxième pédale de coupe.

7. Système chirurgical selon la revendication 6, dans lequel
la première pédale de coagulation et la deuxième pédale de coagulation sont d'une première couleur,
la première pédale de coupe et la deuxième pédale de coupe sont d'une deuxième couleur, différente de la première couleur, et
l'appareil de commande est configuré pour afficher, quand le pied de l'opérateur se rapprochant de l'une de la première pédale de coagulation et de la deuxième pédale de coagulation de pied est détecté par l'un au moins des capteurs, des informations avec la première couleur dans l'une correspondante de la première zone et de la deuxième zone, et
l'appareil de commande est en outre configuré pour afficher, quand le pied de l'opérateur se rapprochant de l'une de la première pédale de coupe et de la deuxième pédale de coupe est détecté par l'un au moins des capteurs, des informations avec la deuxième couleur dans l'une correspondante de la première zone et de la deuxième zone.

8. Système chirurgical selon l'une quelconque des revendications 1 à 7, dans lequel
l'appareil de commande est configuré pour afficher, quand l'une des première à troisième pédales de pied est actionnée, des informations dans l'une des première à troisième zones correspondante, les informations étant affichées dans une forme d'affichage différente d'une forme d'affichage dans un cas où le pied de l'opérateur se rapproche de la pédale de pied.

9. Système chirurgical selon l'une quelconque des revendications 1 à 8, dans lequel
l'un des manipulateurs supporte un instrument chirurgical de remplacement (201c),
la section de pédale d'actionnement inclut en outre une quatrième pédale de pied (25) pour remplacer l'un du premier instrument chirurgical à actionner par la première manette d'actionnement et du deuxième instrument chirurgical à actionner par la deuxième manette d'actionnement avec l'instrument chirurgical de remplacement, et
l'interface utilisateur graphique inclut une quatrième zone (324) qui est agencée entre la première zone et la deuxième zone et affiche des informations sur l'instrument chirurgical de remplacement.

10. Système chirurgical selon l'une quelconque des revendications 1 à 9, dans lequel
la section de pédale d'actionnement inclut en outre une cinquième pédale de pied (23) pour exécuter une fonction de débrayage, et
l'interface d'utilisateur graphique inclut une cinquième zone (325) qui affiche des informations sur la fonction de débrayage.

11. Système chirurgical selon l'une quelconque des revendications 1 à 10, dans lequel
l'appareil de commande est configuré pour afficher, quand la troisième pédale de pied est actionnée, l'interface d'utilisateur graphique incluant l'affichage d'un niveau (328) pour l'endoscope, empiétant sur l'image capturée par l'endoscope.

12. Système chirurgical selon l'une quelconque des revendications 1 à 11, dans lequel
chacune des première à troisième zones de l'interface d'utilisateur graphique affiche un état d'actionnement d'une pédale de pied correspondante.

13. Procédé d'affichage d'informations dans un système chirurgical (400),
le système chirurgical incluant : des manipulateurs (201) qui supportent respectivement un endoscope (201d), un premier instrument chirurgical (201a), et un deuxième instrument chirurgical (201b) ; un appareil de commande distant (100) qui inclut un dispositif d'affichage (3) configuré pour afficher une image capturée par l'endoscope, une première manette d'actionnement (la) pour actionner le premier instrument chirurgical, et une deuxième manette d'actionnement (1b) pour actionner le deuxième instrument chirurgical ; une section de pédales d'actionnement incluant des pédales de pied ; et un appareil de commande configuré pour commander au moins le dispositif d'affichage,
la section de pédales d'actionnement inclut des capteurs de type sans contact (26) qui détectent la présence d'un pied d'un opérateur se rapprochant des pédales de pied respectives,
les pédales de pied incluent une première pédale de pied (21b, 22b) pour exécuter une fonction du premier instrument chirurgical, une deuxième pédale de pied (21a, 22a) pour exécuter une fonction du deuxième instrument chirurgical, et une troisième pédale de pied (24) pour exécuter une fonction de l'endoscope, le procédé comprenant :
afficher une surface d'utilisateur graphique (32) incluant une première zone (321) qui affiche des informations sur le premier instrument chirurgical, une deuxième zone (322) qui affiche des informations sur le deuxième instrument chirurgical, et une troisième zone (323) qui affiche des informations sur l'endoscope ; et
changer, quand le pied de l'opérateur se rapprochant de l'une au moins des première à troisième pédales de pied est détecté par l'un au moins des capteurs, une forme d'affichage de l'une au moins des première à troisième zones correspondante.

14. Procédé selon la revendication 13, dans lequel
changer la forme d'affichage de l'une au moins des zones correspondante inclut au moins un changement de couleur d'affichage, un affichage inversé, et un changement d'affichage d'une ligne de cadre de l'une au moins des zones correspondante.

15. Procédé selon la revendication 13, dans lequel
l'affichage, quand la troisième pédale de pied est actionnée, de l'interface d'utilisateur graphique inclut l'affichage d'un niveau (328) pour l'endoscope, en empiétant sur l'image capturée par l'endoscope.
